# EUROPEAN PATENT APPLICATION

(11) **EP 3 252 469 A1**
(43) Date of publication of application: **06.12.2017**
(21) Application number: 16743178.2
(22) Date of filing: 19.01.2016
(51) Int. Cl.: G01N 33/53, C12Q 1/68, G01N 21/64, G01N 33/48, G01N 33/536, G01N 33/574, B01L 3/00, G02B 21/34, G02B 21/36

(54) **METHOD FOR SIMULTANEOUS DETECTION OF BLOOD CELLS HAVING INTERACTING MOLECULES**

(30) Priority: 29.01.2015 JP 2015015174
(71) Applicant: Konica Minolta, Inc., Tokyo 100-7015 (JP)
(72) Inventor: MASUBUCHI, Manami, Tokyo 100-7015 (JP); CHIYODA, Tsuneko, Tokyo 100-7015 (JP)
(74) Representative: Henkel, Breuer & Partner
(86) International application number: PCT/JP2016/051428
(87) International publication number: WO 2016/121574

(57) **Abstract**

An object of the present invention is to provide a method of simultaneously detecting information that relates to first and second target cells for the purpose of measuring the expression state (presence/absence of expression, expression level) based on the same standard for both a molecule (molecule A) expressed on the first target cells and a molecule (molecule B) expressed on the second target cells, which molecules interact with each other.

To accomplish this object, the method of the present invention includes: adding erythrocyte-removed blood to a cell-spreading substrate; staining the respective markers and nuclear markers of the first and second target cells to acquire fluorescence images thereof; identifying the first and second target cells from the fluorescence images; and detecting molecule A-expressing first target cells and molecule B-expressing second target cells, whereby the expression-level information can be analyzed simultaneously for the molecule A and the molecule B.

SEQUENCE LISTING

<110>  Konica Minolta, Inc.

<120>  Method for Simultaneously Analysing Blood Cells Having Interactive
Molecules

<130>  B36072WO01

<150>  JP2015-15174
<151>  2015-01-29

<160>  6

<170>  PatentIn version 3.5

<210>  1
<211>  20
<212>  DNA
<213>  Artificial Sequence

<220>
<223>  Fw primer for PD-L1

<400>  1
gccgaagtca tctggacaag                                                   20


<210>  2
<211>  20
<212>  DNA
<213>  Artificial Sequence

<220>
<223>  Rv primer for PD-L1

<400>  2
tctcagtgtg ctggtcacat                                                   20


<210>  3
<211>  20
<212>  DNA
<213>  Artificial Sequence

<220>
<223>  probe with FAM

<400>  3
caccaccacc aattccaaga                                                   20


<210>  4
<211>  20
<212>  DNA
<213>  Artificial Sequence

<220>
<223>  Fw primer for PD-1

<400>  4
atagaaccac agggaagggg                                                   20

<210>  5
<211>  20
<212>  DNA
<213>  Artificial Sequence

<220>
<223>  Rv primer for PD-1

<400>  5
ttatttaaag gggttggccg                                                    20


<210>  6
<211>  20
<212>  DNA
<213>  Artificial Sequence

<220>
<223>  probe with HEX

<400>  6
gggtcctcac ccccacgtca                                                    20

## Description

### TECHNOLOGICAL FIELD

The present invention relates to a method of simultaneously detecting first and second target cells.

### BACKGROUND

Blood usually contains hemocytes such as erythrocytes and leukocytes (neutrophils, eosinophils, basophils, lymphocytes and monocytes) and may also contain rare cells such as circulating tumor cells (CTCs), circulating vascular endothelial cells (CECs), circulating vascular endothelial progenitors (CEPs) and other progenitor cells. Further, a cultured cell population may contain stem cells, specific differentiated cells and other characteristic cells.

For example, CTCs are cells that are found in the blood of patients suffering from breast cancer, lung cancer, prostate cancer, pancreas cancer or the like, and CTCs have been drawing attention since they provide clinically important information as the number thereof reflects the metastatic properties of cancer and the like. However, the density of CTCs in blood is extremely low (when the density is low, 1 to 10 CTCs or so per 10 mL of whole blood), and this makes it difficult to detect and count CTCs.

For the detection of rare cells and characteristic cells such as CTCs, methods of detecting CTCs and the like in blood by performing nuclear staining, fluorescent staining or the like are known.

In this process, for the purpose of creating a condition where microscopic observation and staining can be easily performed, for example, a system of spreading cells in a cell suspension using a cell-spreading device that is produced by arranging a flow channel-forming frame on a substrate comprising, on its surface, microchambers or grooves capable of storing and retaining cells is employed (see, for example, Patent Document 1).

To perform profiling of biomarkers expressed on CTCs using such a means is also important for advancing the research of CTCs from various standpoints of, for example, elucidating the mechanism of metastasis through comparison with the profiling of primary cancer biomarkers, assessing useful anti-cancer agents (molecular target drugs) and specifying CTC subpopulations (CTCs having the properties of epithelial cells, mesenchymal cells, stem cells or the like).

In recent years, therapeutic drugs targeting leukocytes have been actively developed in the field of cancer immunotherapy. Leukocytes (T-cells) can specifically attack cancer cells based on the immune system; however, it is known that the immunity is suppressed by interactions between the PD-1 molecules expressed on leukocytes and the PD-L1 molecules expressed on cancer cells (Non-patent Document 1). Accordingly, substances which bind to the PD-1 molecules expressed on leukocytes and thereby inhibit the interactions of the PD-1 molecules with the PD-L1 molecules expressed on cancer cells, for example, therapeutic drugs which contain an anti-PD-1 antibody as an effective ingredient and cancel the immunosuppression caused by the above-described mechanism, have been developed. Conversely, it is also possible to develop a substance which binds to the PD-L1 molecules expressed on cancer cells and thereby inhibits the interactions of the PD-L1 molecules with the PD-1 molecules expressed on leukocytes, for example, a therapeutic drug containing an anti-PD-L1 antibody as an effective ingredient.

However, for a therapeutic drug such as the above-described anti-PD-1 antibody or anti-PD-L1 antibody to be effective, it is a prerequisite that the immunity is in a suppressed state by expression of PD-1 molecules and PD-L1 molecules on leukocytes and cancer cells, respectively. For instance, even when PD-1 molecules are expressed on leukocytes, if PD-L1 molecules were not expressed on cancer cells, immunosuppression would not occur in the first place and leukocytes should thus be able to attack the cancer cells; therefore, rather than a therapeutic drug such as an anti-PD-1 antibody or anti-PD-L1 antibody which is intended to cancel the immunosuppression, for example, an anti-cancer agent or leukocyte immunopotentiator that relates to other action mechanism can be an effective therapeutic drug in such a case. This also applies to those cases where PD-L1 molecules are expressed on cancer cells but PD-1 molecules are not expressed on leukocytes.

In this manner, for a decision of administering a therapeutic drug relating to interactions between two kinds of cells, regardless of which kind of cells a molecule targeted by the therapeutic drug exists on, it is necessary to simultaneously examine the information on the expression of the molecule on both kinds of cells. For example, in order to make an administration decision in the case of using an anti-PD-1 antibody as a therapeutic drug for cancer immunotherapy, not only the information on the expression of a molecule (PD-1) on leukocytes but also the information on the expression of PD-L1 molecule, which interacts with the PD-1 molecule, on cancer cells are required, and it has been revealed that no therapeutic effect is attained by administering an anti-PD-1 antibody to a cancer patient without expression of the PD-L1 molecule on the cancer cells (Non-patent Document 2).

Conventionally, methods of identifying and analyzing rare cells such as CTCs from a variety of cells contained in a blood sample are known; however, these methods do not employ such a system that also analyzes cells other than rare cells, such as leukocytes, simultaneously with rare cells. For example, Patent Document 2 describes a method of detecting CTCs in a blood sample derived from a patient of cancer or the like by processing fluorescence images in which nuclei and a prescribed cell marker are stained and morphological images of cells and their nuclei. In this method, the total number of nuclear stained cells is measured regarding that the nuclear stained cells constitute CTCs and non-CTCs (leukocytes) as a whole and the cells showing the staining pattern of a prescribed cell marker are identified and counted as CTCs and, based on the results thereof, the ratio of the number of CTCs with respect to the total number of cells and the CTC concentration in a blood sample are calculated to analyze the CTCs contained in the blood sample derived from a patient of cancer or the like. However, Patent Document 2 does not concretely mention at all with regard to also analyzing (not just identifying and counting) non-CTCs along with CTCs, particularly simultaneously analyzing both CTCs expressing a prescribed molecule on their surface and leukocytes expressing other prescribed molecule on their surface, which prescribed molecules interact with each other, based on the same standard.

Further, although it is theoretically possible to simultaneously identify, count and analyze both CTCs (expressing a prescribed molecule) and leukocytes (expressing other prescribed molecule) using an existing flow cytometer, since the number of CTCs contained in a blood sample is extremely small, detection failure may occur and it is thus difficult to accurately perform a CTC-related analysis using this system.

Incidentally, the present applicant invented a method of sequentially feeding a cell suspension and required reagents to a flow channel using the above-described cell-spreading device and thereby efficiently amplifying the nucleic acids contained in the cells recovered in each microchamber by PCR or the like, and has previously filed a patent application for an invention relating to this method and the like (Application No.: Japanese Patent Application No. 2014-191129, Filing Date: September 19, 2014).

### DESCRIPTION OF THE RELATED ART

### PATENT DOCUMENTS

[Patent Document 1] WO 2014/061675
[Patent Document 2] Japanese Translated PCT Patent Application Laid-open No. 2013-508729

### NON-PATENT DOCUMENTS

[Non-patent Document 1] Nat Rev Cancer. 2012 Mar 22; 12(4): 252-64
[Non-patent Document 2] N Engl J Med 2012; 366:2443-2454 June 28, 2012

### SUMMARY OF THE INVENTION

### PROBLEMS TO BE SOLVED BY THE INVENTION

In order to solve the above-described problems, the present inventors discovered the significance of measuring the expression state (presence/absence of expression and expression level) based on the same standard for both a molecule expressed on leukocytes and a molecule expressed on CTCs (cancer cells), which molecules interact with each other. Therefore, an object of the present invention is to provide a method of simultaneously detecting information that relates to two different kinds of cells.

### TECHNICAL SOLUTION

The present inventors intensively studied to solve the above-described problems and consequently discovered that, in a method of identifying first and second target cells through staining of their cell markers and nuclei, by detecting a molecule A existing on cells identified as the first target cells and a molecule A-binding molecule B existing on cells identified as the second target cells, it is possible to measure the expression state of the molecule A and that of the molecule B based on the same standard and to analyze the expression-level information for each of the molecules A and B, thereby completing the present invention.

That is, one aspect of the present invention provides a method of simultaneously detecting blood cells, the method comprising the following steps (a) to (f):
(a) removing erythrocytes from blood of a patient (sample treatment step);
(b) adding a prescribed amount of the blood treated in the step (a) to a cell-spreading substrate to arrange first and second target cells on the substrate (cell spreading step);
(c) staining the respective cell markers and nuclear markers of the first and second target cells with phosphors (staining step);
(d) acquiring fluorescence image(s) of the cell markers and nuclear markers thus stained in the step (c) (image acquisition step);
(e) identifying the first and second target cells from the fluorescence images of the cell markers and nuclear markers thus acquired in the step (d) (identification step); and
(f) detecting cells expressing a specific molecule (molecule A) among those cells identified as the first target cells in the step (e) as well as cells expressing other specific molecule (molecule B), which interacts with the molecule A, among those cells identified as the second target cells in the step (e) (detection step).

### ADVANTAGEOUS EFFECTS OF THE INVENTION

According to the method of the present invention, the molecular expression information of the first target cells and that of the second target cells can be obtained based on the same standard. This is expected to improve the reliability of a decision of administering a drug for treatment such as cancer immunotherapy, which is required to be determined based on the information relating to both of the first and second target cells. Particularly, when it is revealed as a result of examination that the molecule A expressed on the first target cells (e.g., PD-L1 expressed on CTCs) and the molecule B expressed on the second target cells (e.g., PD-1 expressed on leukocytes), which molecules interact with each other, are both present, since interactions between the molecules A and B (immunosuppression in the above-described case) are presumed to occur, it can be judged necessary to address the situation with a drug or the like. Meanwhile, when either the molecule A or B does not exist, since no interaction is presumed to occur, it can be judged unnecessary to administer a drug or the like which acts on either of the molecules for the inhibition of interaction (since administration of such a drug does not show any effect, administration of other drug should be considered if possible).

### BRIEF DESCRIPTION OF THE DRAWING

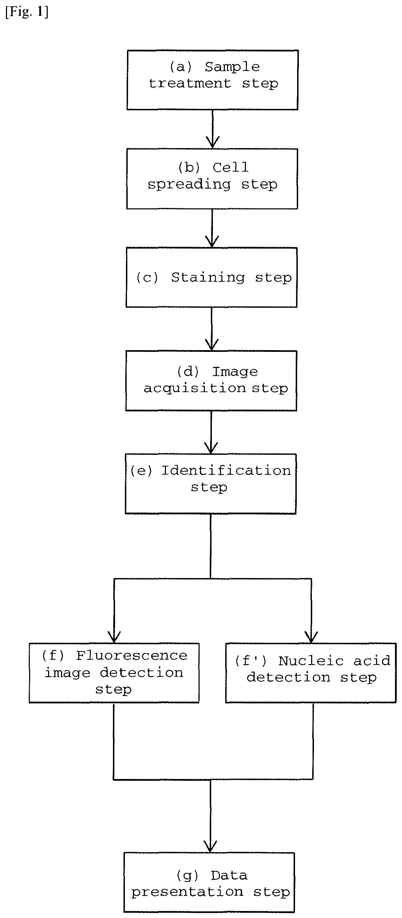

[Fig. 1] Fig. 1 is a flow chart which illustrates one embodiment of the method of simultaneously detecting first and second target cells according to the present invention.

### MODE FOR CARRYING OUT THE INVENTION

Embodiments of the present invention will now be described; however, the present invention is not restricted thereto.

### -Method of Simultaneously Detecting First and Second Target Cells-

The method of simultaneously detecting first and second target cells according to the present invention comprises, at least: (a) the sample treatment step; (b) the cell spreading step; (c) the staining step; (d) the image acquisition step; (e) the identification step; and (f) the detection step, and may further comprise, as required, (g) the data presentation step. These steps of the detection method can be performed by, for example, the procedures in accordance with the flow chart shown in Fig. 1.

### (Target Cells)

In the present invention, there are two kinds of target cells which are, at least, not erythrocytes. The first target cells are, for example, rare cells that include CTCs, CECs, CEPs and other progenitor cells, and the second target cells are, for example, leukocytes (lymphocytes).

### (Molecules of Interest)

The molecules of interest in the present invention are two kinds of molecules (proteins, particularly antigens that can be immunostained in the first embodiment) which are each expressed on the surfaces of the first target cells and the surfaces of the second target cells and interact with each other. In the present specification, the specific molecule expressed by the first target cells is denoted as "molecule A", while the specific molecule expressed by the second target cells is denoted as "molecule B".

The molecules of interest are not particularly restricted and may be selected taking into consideration the use of the quantification and detection method of the present invention, such as pathological diagnosis. Examples of typical molecules of interest in the present invention include PD-L1 which is expressed on CTCs and interacts with PD-1, and PD-1 which is expressed on leukocytes (T-cells). These molecules are known to suppress immunity by interacting with each other.

### (Blood)

The term "blood" used herein is intended to encompass arbitrary components of whole blood, including gerythrocytes, leukocytes, platelets, endothelial cells, mesothelial cells and epithelial cells. The term "blood" also encompasses plasma components, such as proteins, lipids, nucleic acids and carbohydrates; and other arbitrary cells that potentially exist in blood due to pregnancy, organ transplant, infection, trauma or disease, for example, rare cells such as CTCs, CECs, CEPs and other progenitor cells.

### (Leukocytes)

The term "leukocytes" used herein means leukocytes or cells of the hematopoietic system that are not reticulocytes or platelets. The term "leukocytes" encompasses lymphocytes such as natural killer cells ("NK cells"), B-cells and T-cells, as well as phagocytes and mast cells, such as monocytes, macrophages and granulocytes.

### (Erythrocytes)

The term "erythrocytes" used herein means erythrocytes, particularly non-nucleatederythrocytes.

### (Granulocytes)

As described, the term "granulocytes" used herein means a group of cells included in leukocytes and encompasses neutrophils, eosinophils and basophils.

The method of simultaneously detecting blood cells according to the present invention can take two embodiments depending on the mode of performing the step (f): detection step. In the first embodiment, the molecule A-expressing first target cells and the molecule B-expressing second target cells are detected based on fluorescent staining of the respective molecules A and B. In the second embodiment, a substrate comprising microchambers is used as a cell-spreading substrate, and the molecule A-expressing first target cells and the molecule B-expressing second target cells are detected based on nucleic acid amplification reactions of the respective genes of the molecules A and B in the microchambers.

### -First Embodiment-

### -Step (a): Sample Treatment Step-

The sample treatment step (a) is, at least, the step of removing erythrocytes from a patient's blood. When the preparation of a sample used in the present invention, it is appropriate to perform the removal of erythrocytes and other necessary pretreatments in advance. The term "patient" used herein encompasses not only a subject who is suffering from cancer or the like but also a subject who is suspected of having cancer or the like.

### (Anticoagulation Treatment)

The blood (whole blood) collected and taken out of the body coagulates over time once it directly comes into contact with the air, making it impossible to recover and observe the cells contained therein. Thus, it is preferred that the collected blood be immediately subjected to an anticoagulation treatment.

A variety of anticoagulants for whole blood are known, and any of them can be used in accordance with general conditions such as concentration and treatment time. Examples of such anticoagulants include those of a type which, as represented by ethylenediamine tetraacetic acid (EDTA) and citric acid (including salts such as sodium salt), binds to calcium ions by chelating effect and thereby removes the calcium ions from the reaction system to inhibit coagulation; and those of a type which, as represented by heparin, forms a complex with antithrombin III in plasma and thereby suppresses the thrombin production to inhibit coagulation. A blood collection tube in which any of such anticoagulants has been stored in advance may be utilized as well.

Further, as required, the cells may also be subjected to, for example, a fixation treatment for delaying autolysis and decomposition of the cells and thereby maintaining the form and antigenicity of the cells; a permeabilization treatment for improving the permeability of cell membranes and thereby facilitating permeation of substances (e.g., a fluorescently labeled antibody which targets an antigen in the cells, or a nuclear staining agent) from outside of the cells into the cells; and a blocking treatment not only for inhibiting adsorption of the cells to those parts on a cell-spreading substrate other than the parts involved in the immobilization of the cells such as microchambers, but also for inhibiting non-specific adsorption of a fluorescently labeled antibody to those parts other than the antigen of interest in immunostaining performed as required. In cases where such fixation treatment, permeabilization treatment and blocking treatment are not performed simultaneously with the sample treatment step, these treatments may be performed simultaneously with, for example, the staining step as described below.

### (Removal of Erythrocytes, Granulocytes, etc.)

For observation of spread cells, it is required that the subject cells be spread in the observation region such that the cells do not overlap with each other. For this purpose, it is necessary to remove erythrocytes that account for about 96% of the tangible components in blood from the sample. By removing erythrocytes, unnecessary overlapping of cells is inhibited and the subject cells are thus efficiently spread, enabling to detect rare cells without a reduction in the detection sensitivity.

The means for removing erythrocytes is not particularly restricted and, for example, erythrocytes may be hemolyzed using ammonium chloride or the like. Alternatively, erythrocytes may be separated from other cells by centrifugation and then a cell fraction containing at least the first and second target cells may be purified. These methods are known and can be performed using an appropriate centrifuge under appropriate centrifugal conditions.

Density gradient centrifugation is known as a method by which the blood components containing various cells can be fractionated based on the specific gravity. Particularly, for the purpose of removing erythrocytes contained in a large amount in blood, preferably granulocytes as well, and then subjecting only a cell fraction(s) containing the first and second target cells to the cell spreading step, it is preferred to employ density gradient centrifugation.

A separation medium used in density gradient centrifugation may be any medium as long as it has a specific gravity appropriate for the fractionation of blood cells and is prepared to have such osmotic pressure and pH that do not cause cell destruction and, for example, a commercially available sucrose solution such as Ficoll (registered trademark) or Percoll (registered trademark) can be used. By performing a density gradient centrifugation treatment with the specific gravity of the separation medium being adjusted to be lower than that of erythrocytes but higher than that of leukocytes, the blood sample can be separated into at least two layers of "a fraction containing a large amount of erythrocytes" and "a fraction containing a large amount of cells other than erythrocytes".

For example, in one mode of the present invention, by adjusting the specific gravity of the separation medium to be 1.119 or less, preferably 1.113 or less, more preferably 1.085 or less, the erythrocyte contamination ratio in a fraction containing a large amount of nucleated cells other than erythrocytes (i.e., the first and second target cells) can be reduced to 2 to 6% or lower.

Further, in one mode of the present invention, among leukocytes, lymphocytes such as T-cells are used as the second target cells and, in this case, the second target cells (lymphocytes) and the first target cells (CTCs) can be separated from erythrocytes and leukocytes other than the second target cells (e.g., granulocytes) by adjusting the specific gravity of the separation medium to be about 1.077.

Further, a combination of two or more separation media having different specific gravities may be used as well. For example, by using a separation medium having a specific gravity of about 1.077 in combination with a separation medium having a specific gravity of about 1.113, a fraction containing a large amount of the second target cells and a fraction containing a large amount of granulocytes can be obtained.

### -Step (b): Cell Spreading Step-

The cell spreading step (b) is the step of adding a prescribed amount of the blood treated in the sample treatment step (a) to a cell-spreading substrate to arrange the first and second target cells on the substrate.

### (Cell-spreading Substrate)

The cell-spreading substrate in the present invention typically corresponds to a substrate (flow channel substrate) of a device used for the below-described cell observation method and forms a flow channel in combination with a flow channel-forming member. The cell-spreading substrate is used in such a manner that, by introducing a cell suspension to the flow channel, cells contained in the cell suspension are spread on the surface of the cell-spreading substrate.

In the present invention, it is preferred that fine chambers (microchambers) or grooves be formed on the cell-spreading substrate so that the cells contained in the cell suspension can be recovered in a state suitable for fluorescence observation or nucleic acid analysis.

When the cell-spreading substrate is in the mode of having grooves, it is preferred that the width and depth of the grooves be both set in a range of 1 to 100 µm. With the dimensions being this range, cells can be certainly spread in the grooves. The cross-sectional shape of the grooves is not particularly restricted; however, the grooves preferably have a V-shaped cross-section since this allows cells to be densely arranged therein. Other than a V-shape, for example, the grooves may also have a shape in which only one side is obliquely inclined, a shape in which the top and bottom parts are rounded, or a U-shape. It is preferred that the grooves have the same dimensions for the width and the depth. By setting the groove dimensions in this manner, not only it is made difficult for the cells once entered the grooves to move but also interception of other cells by the cells in the grooves can be inhibited.

The grooves can be processed by a known microprocessing technology and, for example, a commercially available prism sheet can also be used instead. Usually, a prism sheet has plural grooves of the same size that are arranged in the form of parallel rows on upper surface of a flat plate, and prism sheets whose groove width and depth are in the above-described range are commercially available.

When the cell-spreading substrate is in the mode of having microchambers, the shape of the microchambers is not particularly restricted; however, it is preferably, for example, an inverted truncated cone shape with flat bottom and tapered side. The bottom diameter and depth of the microchambers can be adjusted as appropriate such that cells can be recovered and stored therein in a number appropriate for nucleic acid analysis and stained image observation. For example, it is preferred that the bottom diameter and the depth be both set in a range of 20 to 500 µm so that 1 to 100 cells can be accommodated per microchamber. It is noted here that various cells contained in blood (excluding erythrocytes) generally have a diameter of 5 to 100 µm, and rare cells such as CTCs are said to have a diameter of 10 to 100 µm or so. Further, in cases where a laser diode is used as a light source of excitation light in the observation of a fluorescently stained image, the irradiation area (spot) has, for example, an elliptical shape with a major axis of about 100 µm and a minor axis of about 10 µm. It is appropriate to set the bottom size of the microchambers to be larger than the size of this irradiation area of the laser diode such that, when one microchamber is irradiated with light, the light is prevented from being irradiated over its adjacent microchambers and causing plural microchambers to emit fluorescence simultaneously.

The arrangement of such plural microchambers on the surface of the cell-spreading substrate is not particularly restricted; however, in order to increase the cell recovery rate (ratio of the cells recovered in the microchambers with respect to all of the cells in the suspension) as much as possible, it is preferred to adjust the orientation of the microchambers and the gaps between the microchambers. For example, it is preferred that the microchambers be arranged such that, when a cell suspension is fed to the flow channel, cells precipitate in at least one microchamber between an inlet and an outlet.

A variety of substrate materials are well-known in the art, and such materials may contain one or a plurality of, for example, glass elements; organic plastics, such as polycarbonates, polymethyl methacrylates and polyolefins; polyamides; polyesters; silicons; polyurethanes; epoxys; acryls; polyacrylates; polyesters; polysulfones; polymethacrylates; polycarbonates; PEEKs; polyimides; polystyrenes; and fluoropolymers.

The cell-spreading substrate may also be made of a material to which cells easily adhere. In this case, as described above, it is desired to perform a surface treatment with a blocking agent or the like on those parts other than the inner surfaces of the microchambers or the groove of the cell-spreading substrate.

### (Cell Immobilization Method: Material and Structure of Cell-spreading Substrate)

In order to improve the detection efficiency of the target cells such as rare cells, it is required to immobilize the cells, that is, to fix the positions of the cells on the cell-spreading substrate. By immobilizing the cells, it is made easier to specify the positions of the cells to be observed and, as required, the detected target cells can be recovered.

Methods for cell immobilization are generally classified into methods of restricting the range of the cell movement based on the surface structure of the cell-spreading substrate (structural methods) and methods of allowing cells to adsorb and be thereby fixed through physical interactions caused by the surface properties of the cell-spreading substrate (interactive methods).

Examples of the structural methods for immobilization include a method of, as described above, forming plural microchambers or grooves on the surface of the cell-spreading substrate and thereby restricting the movement of the cells only to the inside of the chambers or grooves.

Examples of the interactive methods for immobilization include modification of the inner surfaces, particularly the bottom surfaces of the microchambers or the inside of the grooves to have such properties that allow cells to easily adsorb through interactions, that is, to be cell-adhesive. Specific examples thereof include a method of preparing the inner wall surfaces of the microchambers or grooves or the entirety of the cell-spreading substrate from a hydrophobic material to which cells easily adsorb through physical (intermolecular) interactions, such as a plastic or glass (e.g., polystyrene, polymethyl methacrylate or polycarbonate). In cases where the substrate is entirely made of such a substance, in order to prevent the cells from adsorbing thereto in the middle of transfer and interfering with the recovery of themselves in the microchambers or grooves, it is preferred to perform a blocking treatment on the cells themselves so as to make the cells lose their adsorption capability, or to allow the cells to retain the easily-adsorbing properties only for the necessary parts of the inner wall surfaces and the like of the microchambers or grooves.

Examples of the interactive methods also include a method of coating the inner wall surfaces of the microchambers or grooves with a molecule which improves the cell adhesion, such as poly-L-lysine. It is also possible to employ a method of subjecting the parts of the cell-spreading substrate surface other than the inside of the microchambers or grooves to, for inhibition of cell adsorption thereto, a treatment with a blocking agent or moderate hydrophilization by a UV-ozone treatment in which the cell-spreading substrate is irradiated with UV under atmospherics or an oxygen-plasma treatment. By keeping the inside of the microchambers or grooves in a state where a material to which cells easily adhere is exposed, the cells are allowed to adsorb thereto and prevented from moving back out of the microchambers or grooves due to the flow of the cell suspension.

### (Pre-wetting Liquid Feeding Step)

In the cell spreading step (b), prior to feeding the cell suspension, the step of feeding a pre-wetting liquid may be incorporated for the purpose of, for example, improving the wettability of the surface of the cell-spreading substrate. When the cell-spreading substrate is formed from a hydrophobic material such as polystyrene, even if the cell suspension is introduced to the flow channel, air bubbles may remain in the microchambers or grooves due to surface tension, so that the cell suspension cannot be entirely loaded to the microchambers or grooves in some cases. In order to inhibit such a problem, the wettability of the surface of the cell-spreading substrate can be improved by introducing a low-surface-tension organic solvent, such as an aqueous ethanol solution, as a pre-wetting liquid to the flow channel in advance prior to spreading the cell suspension on the surface of the cell-spreading substrate. After such a treatment with a pre-wetting liquid, by introducing physiological saline (e.g., PBS) or pure water to the flow channel so as to replace or wash off the pre-wetting liquid and subsequently introducing the cell suspension, almost the entirety of the cell suspension is brought into the microchambers or grooves and the cells can thus be recovered efficiently.

The pre-wetting liquid is not particularly restricted as long as it is an aqueous solution having a lower surface tension than water and, for example, an aqueous solution containing an alcohol such as ethanol, methanol or isopropyl alcohol, preferably an aqueous ethanol solution having a concentration of 30%(w/v) or lower, or an aqueous solution containing a surfactant, such as Tween (registered trademark) 20, Triton X (registered trademark) or SDS at a concentration of 0.01 to 1%(w/v) can be used.

### -Step (c): Staining Step-

In the staining step (c), the respective marker molecules and nuclear markers of the first and second target cells are stained with phosphors and, in the first embodiment, the molecule A expressed on the first target cells and the molecule B which interacts with the molecule A and is expressed on the second target cells are further stained with phosphors. These staining processes with phosphors can be performed by fluorescent staining using fluorescently labeled antibodies that are each produced from an antibody and a phosphor that are specific to the respective marker molecules of the first and second target cells, fluorescently labeled antibodies that are each produced from an antibody and a phosphor that are specific to the molecule A of the first target cells or the molecule B of the second target cells, and staining agent of a nuclear marker that emits fluorescence.

For example, by introducing a staining liquid, which is an aqueous solution dissolving such fluorescently labeled antibodies and nuclear staining agent, to the flow channel provided on the cell-spreading substrate and thereby filling the flow channel for a prescribed time and bringing the staining liquid into contact with the cells contained in the microchambers or grooves on the cell-spreading substrate, the fluorescently labeled antibodies can be bound to the antigens of the cells.

### (Antigens and Antibodies)

The fluorescently labeled antibodies for the respective markers and molecules of interest (molecule A and molecule B) can be produced by a known method. For example, a desired fluorescently labeled antibody can be produced by using a commercially available monoclonal antibody and fluorescent labeling kit in accordance with the accompanying protocol to allow a prescribed functional group of the monoclonal antibody and that of a fluorescent dye to react and bind with each other in the presence of a prescribed reagent, or by utilizing biotin-avidin binding.

### (Phosphors)

The phosphors used for the immunostaining are not particularly restricted and, for example, a variety of known phosphors (fluorescent dyes) can be suitably used. Taking into consideration the relationships of the fluorescent substances used for the immunostaining of the respective molecules of interest or markers on the first and second target cells in terms of the excitation wavelength and fluorescence wavelength as well as the fluorescence wavelength of the nuclear stain, fluorescent substances each having an appropriate excitation wavelength and fluorescence wavelength may be selected and used such that their fluorescences can each be properly measured with the use of a plurality of appropriate cut filters.

In cases where the cell fixation treatment, permeabilization treatment, blocking treatment and the like are not performed in the sample treatment step, these treatments may be performed simultaneously in the staining step as required. That is, it is also possible to introduce an aqueous solution in which a required agent(s) among a fixation agent, a permeabilization agent and a blocking agent is/are dissolved along with the fluorescently labeled antibodies and nuclear staining agent and to thereby fill the flow channel for a prescribed time and allow the aqueous solution to react with the cells.

### (Marker Molecules)

The marker molecules utilized in the present invention for immunostaining of the first and second target cells are arbitrary cell components that exist in a sample and can be identified by a known microscope technology, histological technology or molecular biological technology, and examples of such marker molecules include, but not limited to, surface antigens, transmembrane receptors and co-receptors, polymers such as proteins and carbohydrates that are bound to or aggregated on the surface, and intracellular components such as cytoplasmic components and nuclear components.

Examples of proteins that are expressed on the cell surface include CD45 which is positive on leukocytes but negative on CTCs, and EpCAMs (Epithelial Cell Adhesion Molecules) that are positive on CTCs having properties of epithelial cells, such as MCF-7 which is negative on leukocytes. Examples of proteins that are intracellularly expressed include cytokeratins which are positive in CTCs but negative in leukocytes. In other words, EpCAMs, cytokeratins and the like can be used as the cell markers of CTCs, and CD45 and the like can be used as the cell markers of leukocytes. When immunostaining is performed using the above-exemplified antigens (cell markers) as targets, cells that are detected as CD45-negative and cytokeratin-positive cells can be determined as CTCs, and cells that are detected as CD45-positive and cytokeratin-negative can be determined as leukocytes.

The time of the staining step, that is, the duration of keeping the cells in contact with a fluorescently labeled antibody solution, can be adjusted as appropriate such that the immunostaining is sufficiently performed in accordance with general immunofluorescent staining, and the time of the staining step may usually be set at 5 minutes to half a day or so.

The temperature of the staining step, that is, the reaction temperature, can be adjusted as appropriate such that the immunostaining is sufficiently performed in accordance with general immunofluorescent staining, and the reaction temperature may usually be set at 4 to 37°C or so.

In the present invention, in order to specify that the cells are nucleated cells regardless of whether they are the first target cells or the second target cells, staining of the nuclear markers (nuclear staining) is further performed in combination. For the nuclear staining, it is preferred to use a fluorescent dye (molecule) which intercalates into double-stranded DNA and thereby emits fluorescence. Such a florescent dye is known as "nuclear staining agent" and, for example, a Hoechst dye which can permeate through cell membranes and stain the nuclei of living cells (e.g., Hoechst 33342 or Hoechst 33258) can be used. It is also possible to use DAPI (4',6-diamidino-2-phenylindole) which cannot stain the nuclei of living cells due to the lack of cell membrane permeability but is capable of staining the nuclei of dead cells whose cell membranes are deteriorated and thus permeable.

The immunostaining of the molecules A and B may be performed simultaneously with the immunostaining of the respective cell markers of the first and second target cells, that is, in the mode of multiple staining where the markers and the molecules A and B are stained simultaneously. In this case, a staining liquid which contains fluorescently labeled antibodies corresponding to the molecules A and B and fluorescently labeled antibodies corresponding to the respective cell markers of the first and second target cells as well as a nuclear staining agent may be brought into contact with the cells retained on the cell-spreading substrate.

Alternatively, rather than the simultaneous multiple staining of the respective cell markers and the molecules A and B, it is possible to remove the antibodies staining the respective cell markers and then freshly immunostain and detect the molecules A and B with antibodies that are specific thereto. Conversely, it is also possible to remove the antibodies staining the molecules A and B and then freshly immunostain the respective cell markers.

For example, in the former case, first, immunofluorescent staining of the respective cell markers of the first and second target cells is performed using a staining liquid which contains fluorescently labeled antibodies corresponding to the respective cell markers of the first and second target cells. A nuclear staining agent is further incorporated into this staining liquid so that cell nuclei can also be stained simultaneously. Next, by bringing a dissociation liquid into contact with the cells, the fluorescently labeled antibodies bound to the respective markers of the first and second target cells are dissociated. In this process, fluorescent substances bound in a mode other than antigen-antibody reaction, such as the nuclear staining agent, are not substantially affected by a dissociation agent and thus kept being bound. After the fluorescently labeled antibodies for the markers are dissociated, the respective molecules of interest are immunostained by the same procedure using a staining liquid which contains fluorescently labeled antibodies corresponding to the molecules A and B.

### (Dissociation Agent)

As the dissociation agent, a substance which shows an action of weakening the affinity contributing to the bonds formed by antigen-antibody reaction can be used, and examples thereof include an acid, an alkali, a salt and a surfactant. Thereamong, an acid is preferred because it imposes relatively minor damage to cell membranes and antigens, and the pH of the acid is preferably 1.0 to 6.0. The acid may be an inorganic acid such as hydrochloric acid, sulfuric acid, nitric acid or phosphoric acid, or an organic acid such as carboxylic acid (e.g., formic acid, acetic acid, citric acid or oxalic acid) or sulfonic acid, and the acid may form a salt having a buffering action, such as glycine hydrochloride (pH = 1.5 to 3 or so).

A surfactant has a property of destructing cell membranes (a surfactant is utilized as a permeabilization agent) and thus causes a stronger damage to cells than an acid, and an alkali and a salt cause a stronger damage to antigens than an acid; however, they can also be used as the dissociation agent. As the alkali, for example, sodium hydroxide or a glycine-sodium hydroxide salt (a buffer having a pH of about 11) can be used. As the salt, for example, sodium chloride or ammonium sulfate can be used. As the surfactant, for example, SDS, guanidine hydrochloride or potassium thiosulfate can be used.

The time of the antigen-antibody dissociation step, that is, the duration of keeping the cells in contact with the dissociation liquid, can be adjusted as appropriate in accordance with the type and concentration of the dissociation agent to be used such that a sufficient dissociation effect can be obtained. For example, when an acid having a pH of 1 to 6 is used as the dissociation agent, the time of the antigen-antibody dissociation step may usually be set at 10 seconds to 30 minutes or so. It is noted here, however, that the time of the antigen-antibody dissociation step is preferably adjusted as appropriate such that the reaction is not excessive to such an extent that causes the loss of antigenicity.

After the staining step but before photographing observation images of the thus stained cells in the image acquisition step, as required, unreacted fluorescently labeled antibodies may be removed with an addition of a washing liquid.

### -Step (d): Image Acquisition Step-

In the image acquisition step (d), fluorescence images of the respective cell markers and nuclear markers stained in the staining step (c) are acquired. In the first embodiment, fluorescence images of the molecules A and B can also be acquired in this step. In cases where no such fluorescence image is acquired, the fluorescence intensity is measured separately in the detection step (f) using a scanning-type device or the like.

The fluorescence images can be photographed in the same manner as in a conventional method. For example, for the purpose of photographing fluorescence images of the cell markers of the first target cells, the cell markers of the second target cells and the nuclear markers and, as required, in the first embodiment, for the purpose of further photographing fluorescence images of the molecules A and B, excitation lights each having an appropriate wavelength corresponding to the respective phosphors may be sequentially irradiated while cutting off unnecessary wavelength components using an appropriate filter(s), and the fluorescence emitted from each phosphor may be observed and photographed.

In addition, for the purpose of, for example, verifying the cell morphology, observation and image photographingunder a bright field may also be performed in combination.

The images acquired in this step can be integrated or corrected as required and then used in the subsequent identification step and detection step.

### -Step (e): Identification Step-

In the identification step (e), from the fluorescence images acquired in the image acquisition step (d) for the respective cell markers and nuclear markers, the first target cells (e.g., CTCs) and the second target cells (e.g., leukocytes) are identified.

For example, in one embodiment of the present invention, cells that are determined as leukocyte marker (e.g., CD45)-negative, CTC marker (e.g., cytokeratin)-positive and nuclear marker-positive are identified as CTCs, and cells that are determined as leukocyte marker-positive, CTC marker-negative and nuclear marker-positive are identified as leukocytes. In cases where the sample is expected to contain leukocytes in a greater amount than CTCs (for example, in an amount of 100 times or greater than the amount of CTCs), nuclear marker-positive cells are identified as all cells and, thereamong, those cells that are leukocyte marker-negative and CTC marker-positive can be identified as CTCs and all of other cells can be identified as leukocytes.

### -Step (f): Detection Step-

In the present invention, molecule A-expressing cells among those cells identified as the first target cells in the identification step (e) as well as molecule B-expressing cells among those cells identified as the second target cells in the identification step (e) are detected in the detection step (f).

In the first embodiment, such detection can be performed on the basis of fluorescent staining of the molecules A and B. For example, the molecules A and B are stained in addition to the staining of the respective cell markers and nuclear markers in the staining step (c), and their fluorescence images obtained in the following image acquisition step (d) are superimposed with each other. As a result, in the identification step (e), when the fluorescence of the thus stained molecule A is detected in the region representing the cells identified as the first target cells, these cells can be identified as the first target cells expressing the molecule A and, in the same manner, when the fluorescence of the thus stained molecule B is detected in the region representing the cells identified as the second target cells, these cells can be identified as the second target cells expressing the molecule B.

In cases where no fluorescence image is acquired for the molecules A and B in the step (d), only the fluorescence intensity is measured using a scanning-type device. By comparing the thus obtained measurement data (the operating distance and the fluorescence intensity indicating the presence of the molecule A and/or the molecule B) with the information on the positions of the first and second target cells on the cell-spreading substrate that are specified from fluorescence images, the presence of "molecule A-expressing first target cells" and/or "molecule B-expressing second target cells" can be detected.

In the first embodiment, for example, it is also possible to measure the number of the cells identified in the identification step (e) as the first target cells as well as the number of the cells identified in the detection step (f) as the molecule A-expressing first target cells and to calculate the ratio of the number of the latter cells with respect to the number of the former cells (cell ratio information). In the same manner, it is also possible to measure the number of the cells identified in the identification step (e) as the second target cells as well as the number of the cells identified in the detection step (f) as the molecule B-expressing second target cells and to calculate the ratio of the number of the latter cells with respect to the number of the former cells (cell ratio information).

Moreover, in the first embodiment, by detecting the molecule A-expressing first target cells and the molecule B-expressing second target cells based on fluorescent staining in the same manner as in the first embodiment and further acquiring the positional information of the respective cells in the detection step (f), groups of the cells that are adjacent with each other can be detected as well. For the detection based on the positional information, by performing the cell spreading step (b) using a cell-spreading substrate provided with reference points (reticle marks), information on the positions of cells having the fluorescence of interest can be acquired as coordinates based on the reference points. For example, when the positional information of the fluorescence signals corresponding to the molecule A-expressing first target cells and the molecule B-expressing second target cells are determined as coordinates using the reference points and the thus determined coordinates exist within a certain range, it can be judged that the cells therein are adjacent with each other. The "certain range" (distance used for the judgement) can be set as appropriate based on the diameter, density and the like of the cells of interest.

The detection step (f) may be performed not only to detect the presence of the molecule A-expressing first target cells and the molecule B-expressing second target cells but also to acquire the information on the amounts of the molecules A and B expressed on the respective cells.

For instance, based on the intensity of the fluorescence emitted from the cells identified as the molecule A-expressing first target cells on the above-described superimposed fluorescence images or the like, the intensity of the fluorescence corresponding to the molecule A contained in the region representing the cells identified as the first target cells can be analyzed as an index which quantitatively indicates the expression level of the molecule A. In the same manner, the intensity of the fluorescence corresponding to the molecule B contained in the region representing the cells identified as the second target cells can be analyzed as an index which quantitatively indicates the expression level of the molecule B. In this case, it is necessary to carefully standardize the conditions such as the labeling ratios of the fluorescent dyes labeling the antibodies, the quantum efficiency, the intensities of the excitation wavelengths, the exposure time and discoloration.

### -Second Embodiment-

In the second embodiment, the sample treatment step (a), the cell spreading step (b) and the identification step (e) can be performed in the same manner as in the first embodiment; however, other steps are different in terms of being performed in the same manner as in the first embodiment or modified, depending on whether or not they are combined with the first embodiment.

In cases where other steps are combined with the first embodiment, the staining step (c) can also be performed in the same manner as the above-described corresponding processes in the first embodiment. In the image acquisition step (d), images of the cellular molecules A and B can be acquired in addition to the fluorescence images of the first and second target cells and their nuclei. In the detection steps (f) and (f), detection of the molecule A-expressing cells (first target cells) and the molecule B-expressing cells (second target cells) and analysis of the expression-level information of the molecules A and B can be performed on the basis of not only the above-described fluorescent staining but also the below-described nucleic acid amplification reactions, and the results thereof can be utilized.

In cases where other steps are not combined with the first embodiment, it is not necessary to perform fluorescent staining of the molecules A and B in the staining step (c). Further, in the image acquisition step (d), only fluorescence images of the first and second target cells and their nuclei may be acquired. In the detection step (f), the detection of the molecule A-expressing cells (first target cells) and the molecule B-expressing cells (second target cells) and, as required, the analysis of the expression-level information are performed based on the below-described nucleic acid amplification reactions, instead of based on the above-described fluorescent staining.

### -Step (f): Detection Step Utilizing Nucleic Acid Amplification Reactions (Nucleic Acid Detection Step)-

The detection step based on nucleic acid amplification reactions in the second embodiment (hereinafter, referred to as "the nucleic acid detection step") is performed when a cell-spreading substrate comprising microchambers is used, and the molecules A and B can each be detected and, preferably their expression levels can be quantified in detail, on the basis of the fluorescence emitted by the nucleic acid amplification reactions.

In the second embodiment, the ratio of the number of microchambers in which the presence of the molecule A or B is detected in the nucleic acid detection step (f) with respect to the total number of microchambers containing the respective cells identified as the first or second target cells by fluorescent staining in the identification step (e) can be calculated. In cases where CTCs are used as the first target cells, since CTCs are rare and the number of CTCs recovered in a single microchamber is thus expected to be 1 at the most, the number of microchambers containing the cells identified as CTCs may be regarded as the number of CTCs and, of these microchambers, the number of microchambers confirmed with the fluorescence corresponding to the molecule A may be regarded as the number of molecule A-expressing CTCs.

Such a nucleic acid detection step is typically performed using a flow channel device which comprises: a cell-spreading substrate surface on which microchambers capable of accommodating and retaining cells are formed; and a flow channel-forming member for the formation of a flow channel having a ceiling on the substrate. Specifically, the nucleic acid detection step comprises, as the preparation stage for amplification of the nucleic acids contained in the cells: the below-described nucleic acid amplification reaction reagent-feeding step (which is substantially performed in the above-described cell spreading step (b)); the sealing liquid-feeding step; the nucleic acid amplification reaction progress step; and the measurement step.

A variety of nucleic acid amplification reactions are known and, in the present invention, a method by which the amount of amplified nucleic acids can be understood quantitatively with time, for example, a real-time PCR method such as a TaqMan probe method, an intercalator method or a cycling probe method, is suitably employed.

When a real-time PCR method or the like is employed for the nucleic acid amplification reactions, the expression levels of the molecules A and B can be quantified in detail for each microchamber using the intensity of the fluorescence emitted by the reactions as an index. In this case, by comparing, for each microchamber, the fluorescence intensities of the molecules A and B with the number of the cells identified as the first and second target cells measured in the identification step (e), a more detailed analysis can also be performed using the average expression level per each of these cells as an index.

Meanwhile, in those cases where a nucleic acid amplification reaction other than a real-time PCR with which the expression levels of the molecules A and B cannot be quantified for each microchamber or those cases where a real-time PCR method is employed but detailed quantification for each microchamber is not necessary, the presence of the molecules A and B in each microchamber may be qualitatively detected based on fluorescence.

In addition, by comparing the immunostaining-based analysis results obtained in the first embodiment and the nucleic acid amplification reaction-based analysis results obtained in the second embodiment, the reliability of the measured values relating to the expression-level information of the molecules A and B can be further improved.

Moreover, for the purpose of enabling to clearly distinguish the results obtained in the nucleic acid amplification reaction progress step performed for the detection of the molecules A and B from the results obtained in the staining and image acquisition steps performed for the identification of the first and second target cells, it is preferred that the fluorescences used in the staining step for staining the first and second target cells be different from the fluorescences used in the nucleic acid amplification reaction progress step for detecting the nucleic acid amplification of the molecules A and B. For example, it is preferred to use fluorescent dyes having different maximum fluorescence wavelengths. In cases where the first embodiment and the second embodiment are combined, it is appropriate (i) to perform the nucleic acid amplification reaction progress step in the microchambers after allowing the fluorescently labeled antibodies used for the immunostaining of the molecules A and B to dissociate from the molecules A and B using such a dissociation agent as described above (in this case, there is no problem even if the fluorescent substances used for the immunostaining and the fluorescent substances used for the nucleic acid amplification reaction progress step are of the same kind having similar maximum fluorescence wavelengths), or (ii) that, if the nucleic acid amplification reaction progress step is to be performed in the microchambers with the fluorescently labeled antibodies used for the immunostaining of the molecules A and B being remained, the fluorescent substances used for the immunostaining and the fluorescent substances used for the nucleic acid amplification reaction progress step are of different kinds having different maximum fluorescence wavelengths.

The steps included in the nucleic acid detection step will now be described below.

### (Nucleic Acid Amplification Reaction Reagent-feeding Step)

In the nucleic acid amplification reaction reagent-feeding step, a nucleic acid amplification reaction reagent is fed to the flow channel such that the microchambers are filled with the nucleic acid amplification reaction reagent. The amount of the nucleic acid amplification reaction reagent to be fed can be adjusted as appropriate taking into consideration the total volume of the microchambers such that the amount is enough to fill up at least the microchambers, and the flow channel may also be filled with the nucleic acid amplification reaction reagent.

As the nucleic acid amplification reaction reagent, one which conforms to the nucleic acid amplification reactions to be performed is used. For example, when a PCR method is employed for the nucleic acid amplification reactions, as the nucleic acid amplification reaction reagent, a reagent which contains primers (forward and reverse primers), dNTPs (dATP, dCTP, dGTP and dTTP), heat-resistant Taq DNA polymerase and other required elements such as Mg²⁺ ions and is appropriate for a nucleic acid amplification method using a buffer containing TaqMan probe and the like (e.g., Tris/HCL buffer) can be generally used.

The nucleic acid amplification reaction reagent is preferably a reagent which has an effect of assisting cytolysis and/or an effect of suppressing PCR reaction-inhibiting substances derived from cells, and examples of such a reagent include Mighty Amp (registered trademark) DNA Polymerase Ver.2 (manufactured by Takara Bio Inc.), Allele-In-One Human Blood Purification-Opt Lysis Buffer (manufactured by Cosmo Bio Co., Ltd.), EzWay (TM) Direct PCR buffer (manufactured by TEFCO) and Ampdirect (manufactured by Shimadzu Corporation).

### (Sealing Liquid-feeding Step)

In the sealing liquid-feeding step, by feeding a sealing liquid to the flow channel and thereby washing away the nucleic acid amplification reaction reagent remaining in the flow channel, the flow channel is filled with the sealing liquid such that the nucleic acid amplification reaction reagent filling the microchambers do not interflow between the microchambers. The amount of the sealing liquid to be fed can be adjusted as appropriate taking into consideration the total volume of the flow channel arranged in the upper parts of the whole microchambers such that the amount is enough to fill up the flow channel, particularly such that the sealing liquid can cover the surface of the cell-spreading substrate other than those parts of the microchambers to prevent the interflow of nucleic acid amplification reaction liquid between the microchambers.

It is appropriate that the sealing liquid has a lower specific gravity than the nucleic acid amplification reaction reagent so that, when the sealing liquid is fed, it does not mix with the nucleic acid amplification reaction reagent in the microchambers. As such a sealing liquid, one which is commonly used in a nucleic acid amplification reaction method such as a PCR method can be employed and, for example, mineral oil is preferred.

### (Nucleic Acid Amplification Reaction Progress Step)

In the nucleic acid amplification reaction progress step, inside the microchambers which contain cells (the first target cells and/or the second target cells) and are filled with the nucleic acid amplification reaction reagent and sealed with the sealing liquid, nucleic acid amplification reactions corresponding to the nucleic acid amplification reaction reagent are performed to amplify the nucleic acids contained in the cells. Such a nucleic acid amplification reaction progress step may be performed by carrying out the operations that correspond to the selected nucleic acid amplification reactions. For example, when a real-time PCR method is selected, a cycle consisting of a temperature treatment (about 94°C) of thermally denaturing a template DNA into single strands, a temperature treatment (about 40 to 60°C) of allowing primers to bind to the single strands and a temperature treatment (about 72°C) of further allowing DNA polymerase to bind thereto and thereby initiating and advancing DNA synthesis reaction is repeated for a prescribed number of times (about 30 to 40 cycles), whereby the genes of the molecules A and B are amplified.

### (Measurement Step)

When fluorescence intensity is used as a quantitative index that reflects the expression levels of the molecules A and B (the amounts of the amplified genes after a certain period of time) in the microchambers, the measurement step is performed along with the progress of the nucleic acid amplification reactions and, when fluorescence is used as a qualitative index that reflects the expression of the molecules A and B in the microchambers, the measurement step is performed after the nucleic acid amplification reactions. In the measurement step, the intensity of the fluorescence emitted from each microchamber is measured while irradiating a prescribed excitation light that corresponds to the phosphor in use. For example, it is preferred to measure the intensity of the fluorescence transmitting through a filter at each position (movement distance) while scanning along the arrangement of the microchambers with an optical mechanism (e.g., PMT or PD). The light source of the excitation light or an excitation filter and a fluorescence filter may be changed in accordance with the fluorescence to be measured. A high fluorescence intensity is measured at the position of a microchamber in which cells containing a nucleic acid having a prescribed base sequence are contained and the nucleic acid has been amplified. Further, in the measurement step, as required, a reflected light, a transmitted light, an intrinsic fluorescence or the like may also be measured for acquiring the position of a microchamber.

### (Information Acquisition Step)

The information acquisition step is a step which is performed after the completion of the nucleic acid amplification reactions in place of the above-described measurement step. Similarly to the measurement step, fluorescence images of the cell-spreading substrate are each photographed by irradiating a prescribed excitation light that corresponds to each phosphor used in the nucleic acid amplification reactions for the molecule A or B, and the thus obtained fluorescence images are superimposed with each other to verify the presence or absence of fluorescence in the microchambers, whereby information on the expression of the molecules A and B can be qualitatively acquired. Here, by further overlaying the positional information of the microchambers in which expressions of the molecules A and B are confirmed and the positional information of the microchambers in which the first or second target cells are conformed in the identification step (e), a more detailed information on the expression of the molecules A and B can be acquired.

### -Step (g): Data Presentation Step-

In the present invention, after the detection step (f) in both of the first and second embodiments, based on the results of analyzing the positional information of the molecule A-expressing first target cells and the molecule B-expressing second target cells and/or the expression-level information of the molecules A and B, the data relating to the administration decision of a drug for the treatment or prevention of a disease associated with the interactions between these molecules can be presented.

For example, a substance which inhibits the functions of the molecule A through some kind of interaction (e.g., binding to the molecule A) and can be used as a drug is hereinafter referred to as "drug a" and, similarly, a substance against the molecule B is hereinafter referred to as "drug b". By analyzing the positional information and statistically processing the quantitative results of analyzing plural samples, a threshold value which allows to judge if groups of the molecule A-expressing first target cells and molecule B-expressing second target cells that are adjacent with each other are present in a sufficient number can be determined. Further, by analyzing the expression-level information and statistically processing the quantitative results of analyzing plural samples, a threshold value which allows to judge if the molecules A and B are expressed can also be determined.

In this process, when, as a result of analyzing a blood sample collected from a patient of a certain disease, the number of the groups of the molecule A-expressing first target cells and the molecule B-expressing second target cells that are adjacent with each other is greater than the threshold value and/or the amount of the molecule A expressed on the first target cells and that of the molecule B expressed on the second target cells are each greater than the threshold value, since it is possible that the symptoms of the patient are being affected by the interactions between the molecule A and the molecule B, the drugs a and b capable of inhibiting the interactions are both expected to have efficacy. On the other hand, when the number of the groups of the molecule A-expressing first target cells and the molecule B-expressing second target cells that are adjacent with each other is less than the threshold value and either the amount of expressed molecule A or that of expressed molecule B is also less the threshold value, since no interaction takes place between these molecules and the symptoms of the patient are thus not affected by the interaction, neither of the drugs a and b is expected to show any effect when administered.

Specifically, for example, a blood sample collected from a cancer patient is analyzed; it is judged whether or not PD-L1 as the molecule A and PD-1 as the molecule B are each expressed in an amount greater than the threshold value on CTCs and leukocytes, respectively, and/or whether or not the groups of PD-L1-expressing CTCs and PD-1-expressing leukocytes that are adjacent with each other are present in a number greater than the threshold value; and then, based on whether or not an anti-PD-L1 antibody as the drug a and an anti-PD-1 antibody as the drug b are expected to show efficacy, the data that contribute to the administration decision of these drugs are presented.

In this data presentation step, not only the above-described data based on the results of analyzing the respective expression-level informations of the molecules A and B and the positional information of the first and second target cells, but also data that relate to the prognostic prediction of a metastatic cancer, the decision of therapeutic effects of a drug being administered and the like may be presented based on the results of identifying and counting CTCs in the identification step.

### <Cell Analysis System>

The cell analysis system of the present invention is a system for carrying out the method of simultaneously detecting the first and second target cells according to the present invention in which cell staining and, as required, nucleic acid amplification are performed as described above.

That is, the cell analysis system of the present invention comprises: (A) a means for removing erythrocytes from a patient's blood so as to perform the sample treatment step (sample treatment means); (B) a means for adding a prescribed amount of the blood treated by the sample treatment means (A) to a cell-spreading substrate to arrange the first and second target cells on the substrate so as to perform the cell spreading step (cell-spreading means); (C) a means for staining the respective cell markers and nuclear markers of the first and second target cells with phosphors so as to perform the staining step (staining means); (D) a means for acquiring fluorescence images of the respective cell markers and nuclear markers stained by the staining means (C) so as to perform the image acquisition step (image acquiring means); (E) a means for identifying the first and second target cells from the fluorescence images of the respective cell markers and nuclear markers acquired by the image acquisition means (D) (identification means); and (F) a means for detecting cells expressing a specific molecule (molecule A) among those cells identified as the first target cells by the identification means (E) as well as cells expressing other specific molecule (molecule B) that interacts with the molecule A among those cells identified as the second target cells by the identification means (E) (detection means).

In the first embodiment of the present invention where the detection by the detection means (F) is performed based on fluorescent staining of the respective molecules A and B, the staining means (C) may further comprise a means for staining the molecules A and B with phosphors, and the image acquisition means (D) may further comprise a means for acquiring fluorescence images of the molecules A and B. A means for analyzing the fluorescence images based on the positional information of the molecule A-expressing cells (first target cells) and the molecule B-expressing cells (second target cells) that are acquired by the above-described fluorescent staining and thereby detecting groups of the molecules A-expressing cells and molecule B-expressing cells that are adjacent with each other is further incorporated. It is noted here that, in cases where no fluorescence image is acquired for the molecules A and B, the cell analysis system also comprises: a means for measuring only the fluorescence intensity using a scanning-type device; and a means for comparing the thus obtained measurement data (the operating distance and the fluorescence intensity indicating the presence of the molecule A and/or the molecule B) with the information on the positions of the first and second target cells on the cell-spreading substrate that are specified from fluorescence images and thereby detecting the presence of "molecule A-expressing first target cells" and/or "molecule B-expressing second target cells".

In the second embodiment of the present invention where the detection by the detection means (F) is performed based on nucleic acid amplification reactions of the respective genes of the molecules A and B, the detection means (F) is a means for detecting nucleic acids (amplification products) of the respective genes of the molecules A and B in the microchambers, specifically, a combination of a nucleic acid amplification reaction reagent-feeding means, a sealing liquid-feeding means and a nucleic acid amplification reaction-advancing means with a measurement means or an information acquisition means.

Further, the cell detection system in a preferred embodiment of the present invention also comprises a means for presenting the data relating to the administration decision of a drug for the treatment or prevention of a disease associated with the interactions between the molecules A and B based on the results of analyzing the positional information of the molecule A-expressing first target cells and the molecule B-expressing second target cells that is acquired by the detection means (F) and/or the expression-level information of the molecules A and B (data presentation means).

These means can be constructed by, for example, the below-described liquid feeding mechanism, optical mechanism, data processing mechanism and control mechanism thereof, or by utilizing other appropriate known technology.

The cell analysis system is generally constituted by: a cell analyzer which comprises mechanisms as the respective means described above; a flow channel device; and a reagent container. The flow channel device and the reagent container are set on the cell analyzer (on a flow channel device holder and a reagent container holder inside the cell analyzer, respectively) at the time of operating the system. The cell analyzer can be constructed as an apparatus which comprises all of the above-described means (mechanisms) in an integrated manner, or as a group of plural apparatuses each comprising one or more of the above-described means (mechanisms).

### <Cell Analyzer>

The cell analyzer comprises, at least: a liquid-feeding mechanism for feeding various liquids, such as the cell suspension used in the cell spreading step, the staining liquid used in the staining step and, as required, the nucleic acid amplification reaction reagent and the sealing liquid and the like that are used in the nucleic acid detection step performed in the second embodiment, to a flow channel of the flow channel device; a flow channel device holder which holds the flow channel device used in these steps; a reagent container holder which holds the reagent container; and a control mechanism for controlling the behaviors of various instruments of the cell analyzer. It is preferred that the cell analyzer also comprise, inside the same apparatus as the liquid-feeding mechanism, an optical mechanism for observing fluorescently stained cells recovered on the cell-spreading substrate of the flow channel device and reaction products of amplifying prescribed genes (nucleic acids) contained in the cells and for photographing fluorescence images thereof. In the second embodiment of the present invention, it is preferred that the cell analyzer further comprise, inside the same apparatus as the optical mechanism and the like, a mechanism required for performing the nucleic acid detection step, such as a microchamber temperature control mechanism. If necessary, the cell analyzer can further comprise, inside the same apparatus as the optical mechanism and the like, a sample treatment mechanism which is capable of removing erythrocytes and the like by density gradient centrifugation or the like.

### (Sample Treatment Mechanism)

The sample treatment mechanism is equivalent to a means for removing erythrocytes from blood collected from a patient by lysis, centrifugation or the like and, preferably, unnecessary leukocytes such as granulocytes as well, depending on the embodiment. The sample treatment mechanism may also have functions for performing various treatments that can be performed simultaneously with the sample treatment, such as a cell fixation treatment, a permeabilization treatment and a blocking treatment.

In the case of removing erythrocytes and the like by centrifugation, the sample treatment mechanism can be constructed in accordance with a conventional centrifuge capable of performing a centrifugation treatment by a prescribed method such as density gradient centrifugation. Further, in the case of removing erythrocytes by lysis and also performing, as required, a cell fixation treatment, a permeabilization treatment, a blocking treatment and/or the like, the sample treatment mechanism can be constructed with a pipette, a chip and the like such that a reagent(s) required for blood can be added.

When the sample treatment mechanism is arranged inside the cell analyzer comprising the liquid-feeding mechanism and the like, it is preferred that, for example, a blood collection tube containing blood be set on the cell analyzer such that the sample treatment is performed automatically. Meanwhile, when the treatment of removing erythrocytes by lysis as well as the cell fixation treatment and the like that are performed as required can be done (simultaneously with a blood anticoagulation treatment) by simply adding the patient's blood to a blood collection tube containing the reagents for these treatments or when the size of the cell analyzer comprising the liquid-feeding mechanism and the like is desired to be reduced, the sample treatment mechanism is not required to be integrated into the cell analyzer comprising the liquid-feeding mechanism and the like, and a separate apparatus for sample treatment (e.g., a common centrifuge) may be utilized as required. In such a case, a sample which has been treated in advance is set on the cell analyzer comprising the liquid-feeding mechanism and the like.

### (Liquid-feeding Mechanism)

The liquid-feeding mechanism is a mechanism for sucking and discharging the cell suspension, staining liquid and washing liquid that are stored in the reagent container as well as the nucleic acid amplification reaction reagent, sealing liquid and other reagents that are used as required and feeding these liquids to the flow channel based on the control by the control means.

The liquid-feeding mechanism can be constructed with, for example, a syringe pump, a replaceable chip and an actuator and the like that is movable in the plane and vertical directions with respect to the flow channel device (cell-spreading substrate) for allowing the liquids to be sucked and discharged at arbitrary positions. The syringe pump is capable of sucking and discharging the liquids used in each step at a desired flow rate.

Specifically, a liquid-feeding mechanism which comprises a supply means and a liquid transfer means separately and a flow channel device which comprises a reservoir on the inlet side can be used. In this embodiment, the supply means sucks up a prescribed amount of the liquids (e.g., cell suspension) stored in the reagent container and subsequently discharges the liquids into the reservoir, and these liquids are temporarily retained in the reservoir. The liquid transfer means is connected to an outlet, and the liquids retained in the reservoir are introduced to the flow channel via the inlet at a prescribed flow rate by suction.

Alternatively, a liquid-feeding mechanism which comprises both a supply means and a liquid transfer means together and a flow channel device which comprises a reservoir on the outlet side can be used as well. In this embodiment, the liquid-feeding mechanism sucks up a prescribed amount of the liquids (e.g., cell suspension) stored in the reagent container and subsequently discharges the liquids at the inlet at a prescribed flow rate to directly introduce the liquids to the flow channel. The liquids that pass through the flow channel and are discharged via the outlet can be temporarily retained in the reservoir. After the completion of prescribed treatments by such liquid transfer, the liquid-feeding mechanism sucks up the liquids filling the flow channel via the inlet and then discharges the liquids. The liquid-feeding mechanism may be configured such that it discharges the liquids into a waste liquid storage part of the reagent container.

### (Microchamber Temperature Control Mechanism)

In the second embodiment of the present invention, in cases where, after the completion of the preparation method for nucleic acid amplification by feeding the nucleic acid amplification reaction reagent and the sealing liquid to the flow channel device using the cell analyzer (liquid-feeding mechanism), the subsequent nucleic acid amplification reaction progress step such as PCR is continuously performed with the flow channel device being set on the flow channel device holder, it is preferred that the flow channel device holder comprise a means for controlling the temperature of the cell-spreading substrate, which is required for allowing the nucleic acid amplification reaction to progress in the respective microchambers.

Such a microchamber temperature control means comprises, for example: a temperature control element which performs heating and cooling, such as a Peltier element; and a temperature detection element, and these elements can be arranged at those parts of the flow channel device holder that are in contact with the cell-spreading substrate. The control means, while referring to the temperature of the cell-spreading substrate measured by the temperature detection element, executes temperature control through heating or cooling performed by the temperature control element such that the cell-spreading substrate has a prescribed temperature at a prescribed timing.

When the cell analyzer (liquid-feeding mechanism) does not have such a microchamber temperature control mechanism, the nucleic acid amplification reaction progress step and other steps can also be performed by removing the flow channel device, which has been subjected to the preparation method for nucleic acid amplification, from the cell analyzer and replacing the thus removed device with an apparatus for nucleic acid amplification reactions that is arranged separately from the device.

### (Optical Mechanism)

The optical mechanism comprises members for exciting fluorescent substances and allowing the substances to emit fluorescence, such as a laser diode (LD) used as a light source, a condenser lens, a pin-hole member, a dichroic mirror and a fluorescence filter. The LD is one which conforms to the excitation wavelength of the fluorescence substance to be observed, and plural LDs may be arranged as required such that an appropriate excitation wavelength is irradiated through the dichromic mirror. The fluorescence filter is one which conforms to the fluorescence wavelength of the fluorescence substance used in a fluorescently labeled antibody, and plural fluorescence filters may be arranged as required such that they are used while being switched by a filter exchanger.

The optical mechanism further comprises members for not only enabling to visually observe fluorescently-stained cells and fluorescence-emitting microchambers as the nucleic acid amplification reaction progresses but also photographing the fluorescence images thereof, such as an objective lens, an eyepiece and a CCD camera, and the optical mechanism may further comprise, as required, members for measuring the fluorescence intensity, such as a photomultiplier tube (PMT).

The optical mechanism comprising these members can be constructed in the form corresponding to a fluorescence microscope and is preferably integrated into the cell analyzer along with the liquid-feeding mechanism. In such an embodiment, the image acquisition step and the measurement step included in the nucleic acid detection step can be performed after the cell spreading step and the staining step or, depending on the embodiment, after or while performing the detection step or the nucleic acid detection step (measurement step), so that the observation and photographing of the fluorescence-emitting cells or microchambers can be performed smoothly. Further, it is also possible to arrange the optical mechanism on an apparatus corresponding to a fluorescence microscope that is separate from the apparatus mainly constituted by the liquid-feeding mechanism and to perform the image acquisition step after moving the flow channel device subjected to the cell spreading step and the staining step using the latter apparatus to the former apparatus.

In cases where cells are analyzed using a cell-spreading substrate comprising microchambers, the optical mechanism may further comprise a means for specifying the positions of the microchambers to be observed as well as the positions of the cells contained in the microchambers. For example, by arranging reference points (reticle marks) on the cell-spreading substrate, information on the positions of the microchambers of interest (microchambers emitting the fluorescence used for labeling the target cells, expressed molecules or nucleic acid amplification reaction products thereof) or the target cells themselves can be acquired as coordinates based on the reference points. By utilizing such information based on the reference points, even when the cell-spreading substrate is moved from the cell analyzer to other apparatus for observation and photographing, the microchambers or cells of interest can be immediately observed based on the information.

Further, even without acquiring any image, by irradiating a fluorescent substance with an excitation light emitted from a light source and allowing the optical mechanism to scan on the cell-spreading substrate and thereby measure the intensity of the fluorescence emitted by the fluorescent substance, information on the positions at which the subject that is labeled with the fluorescent substance exists can also be acquired based on the relationship between the moving distance and the fluorescence intensity. Moreover, the position of the microchambers can be specified by utilizing that, when the cell-spreading substrate scanned while being irradiated with a light emitted from a light source, the resulting transmitted light or reflected light or the intensity of the intrinsic fluorescence emitted by the material constituting the cell-spreading substrate differs between the microchambers and other parts. In such an embodiment, the optical mechanism may further comprise a photodetector, such as a photodiode (PD), for measuring the above-described fluorescence, transmitted light, reflected light or intrinsic fluorescence. By integration of the information on the positions of the microchambers with the information on the fluorescence intensity, it can be precisely specified which microchambers contain the cells of a prescribed type (cells having a prescribed antigen and/or gene).

### (Data Processing Mechanism)

The data processing mechanism is a mechanism which is capable of performing integration, correction and the like of the data such as fluorescence images and fluorescence intensity that are obtained by the optical mechanism using the image acquisition means and the detection means, and the data processing mechanism can be generally constructed by a program (software) for data processing such as image processing, a recording medium in which the program is stored in a computer-executable manner, and a computer for executing the program. Such a data processing mechanism may be provided as a separate device (e.g., a personal computer) which is used by being connected to the cell analyzer comprising the liquid-feeding mechanism, the optical mechanism and the like, or may be integrated into the cell analyzer comprising the liquid-feeding mechanism, the optical mechanism and the like.

The data processing mechanism comprises, at least, a function for identifying the first target cells (e.g., CTCs) and the second target cells (e.g., leukocytes) based on the fluorescence images acquired by the image acquisition means and, in the present invention, the data processing mechanism further comprises a function for detecting, among these cells, the molecule A (e.g., PD-L1)-expressing first target cells and the molecule B (e.g., PD-1)-expressing second target cells from the fluorescence images or other data.

The program is preferably one which is capable of automatically executing, for example: a process of integrating, in a single visual field, a fluorescence image representing the fluorescence emitted from the phosphor labeling the cell marker of the first target cells, a fluorescence image representing the fluorescence emitted from the phosphor labeling the cell marker of the second target cells and a fluorescence image representing the fluorescence emitted from the phosphor labeling the nuclear marker; a process of identifying the regions in the thus integrated image where the pixel information (brightness and color) has prescribed characteristics as the first or second target cells; a process of measuring the number of the thus identified cells of each type; and a process of sequentially performing these processes for all of the photographed visual fields.

In the first embodiment of the present invention, the above-described program is preferably also capable of automatically executing, for example: a process of integrating a fluorescence image representing the fluorescence emitted from the phosphor labeling the molecule A and a fluorescence image representing the fluorescence emitted from the phosphor labeling the molecule B in addition to the above-described three kinds of fluorescence images; a process of detecting the regions in the thus integrated image where the pixel information (brightness and color) has prescribed characteristics as the molecule A-expressing first target cells or the molecule B-expressing second target cells; a process of measuring the number of the thus detected cells of each type; and a process of sequentially performing these processes for all of the photographed visual fields.

Simultaneously with the above-described processes, the information on the expression level can be acquired for the molecules A and B based on the fluorescence intensity of the molecule A on the first target cells and that of the molecule B on the second target cells, respectively. Further, based on the positional information of the first and second target cells, groups of the first target cells and the second target cells that are adjacent with each other can also be detected. It is preferred that the program be capable of automatically executing these processes as well.

Meanwhile, in the second embodiment of the present invention, the above-described program is preferably also capable of automatically executing, for example: a process of, on a fluorescence image representing the fluorescence emitted from the phosphors contained in the microchambers in which nucleic acid amplification reactions are performed, detecting the regions where the pixel information (brightness and color) has prescribed characteristics as the microchambers containing the molecule A-expressing first target cells and/or the molecule B-expressing second target cells; a process of measuring the number of the thus detected microchambers; and a process of sequentially performing these processes for all of the photographed visual fields. Further, also in those cases where the data relating to the fluorescence intensity of the molecules A and B, rather than fluorescence images, are acquired by the above-mentioned scanning-type device or the like, it is preferred that the program be capable of automatically executing the same processes as described above, such as the process of detecting the microchambers containing the molecule A-expressing first target cells and/or the molecule B-expressing second target cells. Moreover, as in the first embodiment, the expression-level information and/or the positional information can also be acquired based on fluorescence intensity. When the cells of both types are contained in a single microchamber formed on the cell-spreading substrate (when the fluorescences of both the molecules A and B are detected), it can be regarded that these cells are adjacent with each other; however, a more optimal case is where at least either of the molecule A-expressing first target cells and the molecule B-expressing second target cells exist in a single microchamber in a sufficiently large number.

The data processing mechanism may further comprise a function of analyzing the following information using the results of the above-described identification and detection processes. In the first embodiment of the present invention, the above-described program is preferably capable of, for example, automatically comparing the measured number of the first target cells and that of the second target cells with the measured number of the molecule A-expressing first target cells and that of the molecule B-expressing second target cells and thereby calculating the ratio of the number of the molecule A-expressing first target cells with respect to the total number of the first target cells and the ratio of the number of the molecule B-expressing second target cells with respect to the total number of the second target cells. Further, in the second embodiment of the present invention, the above-described program is preferably capable of, for example, automatically comparing the number of the microchambers containing the first target cells and that of the microchambers containing the second target cells, which are measured from the three kinds of fluorescence images photographed prior to the nucleic acid detection step and then integrated together and the information indicating the positions of the microchambers in the thus integrated image, with the number of the microchambers containing the molecule A-expressing first target cells and that of the microchambers containing the molecule B-expressing second target cells, which are measured from the fluorescence image photographed in the nucleic acid detection step and the information indicating the positions of the microchambers in the fluorescence image, and thereby calculating the ratio of the number of the microchambers containing the molecule A-expressing first target cells with respect to the total number of the microchambers containing the first target cells and the ratio of the number of the microchambers containing the molecule B-expressing leukocytes with respect to the total number of the microchambers containing the second target cells.

It is preferred that the data processing mechanism further comprise, as a data presentation means, a function of, on the basis of the above-described analysis results, presenting the data that relate to the administration decision of a drug for the treatment or prevention of a disease associated with the interactions between these molecules. For example, a threshold value for determining that the molecule A is expressed on CTCs or that a blood sample derived from a patient contains molecule A-expressing CTCs is recorded in advance and, when this threshold value is compared with a measured value of the expression-level information obtained in the detection step and the measured value is not smaller than the threshold value, the data presentation means may present a judgement result that a substance which inhibits the functions of the molecule A and can be used as a drug (the above-described drug a) and a substance which inhibits the functions of the molecule B and can be used as a drug (the above-described drug b) are both effective for the treatment or prevention of the patient's disease. In the same manner, the data presentation means may also present a judgement relating to the effectiveness of the drugs a and b as a result of comparing the number of groups of the molecule A-expressing first target cells and the molecule B-expressing second target cells that are adjacent with each other, which number is determined based on the positional information obtained in the detection step, with a threshold value relating thereto.

### (Control Mechanism)

The control mechanism is a mechanism which is connected to the various instruments of the cell analyzer and capable of controlling the behaviors of the instruments such that a cell detection method can be carried out in accordance with prescribed steps and procedures. The control mechanism can be generally constructed by a program for such control, a recording medium in which the program is stored in a computer-executable manner, and a computer capable of executing the program. Such a control means may be provided as a separate device (e.g., a personal computer) which is used by being connected to the cell analyzer comprising the liquid-feeding mechanism, the optical mechanism and the like or, as in the case of a microcomputer, may be integrated into the cell analyzer comprising the liquid-feeding mechanism, the optical mechanism and the like. The control program may be stored in a computer's built-in recording medium, or may be put in a state where it can be utilized by a personal computer through a network or removable recording medium.

The control program enables to automate the operations relating to the cell-spreading means (step), the staining means (step), the image acquisition means (step) and the like. For example, the control program enables to operate the liquid-feeding mechanism in such a manner that the cell suspension in the cell spreading step and the staining liquid in the staining step are fed in accordance with a prescribed time schedule and to operate the optical mechanism in such a manner that prescribed excitation lights corresponding to the phosphors contained in the staining agent are irradiated and fluorescence images of the phosphors are photographed. In the second embodiment of the present invention, by the control program, for example, the operations of the liquid-feeding mechanism for feeding the nucleic acid amplification reaction reagent, the sealing liquid and the like in the nucleic acid detection step in accordance with a prescribed time schedule, the operations of the microchamber temperature control mechanism for heating or cooling the microchambers in accordance with the nucleic acid amplification reactions, and the operations of the optical mechanism for irradiating prescribed excitation lights corresponding to the phosphors of fluorescently-labeled nucleic acid amplification probes and photographing fluorescence images thereof so as to detect the fluorescences emitted by nucleic acid amplification products can be automatically performed.

In addition, it is preferred that the control mechanism further comprise: functions of recording the data obtained in relation to cell staining and nucleic acid amplification (e.g., fluorescences relating to the identification of the target cells and the detection of the genes of the molecules of interest, and the transmitted light, reflected light or intrinsic fluorescence relating to the specification of the microchamber positions that is optionally performed) and photographed fluorescence images, reading out the data and images in an appropriate step and delivering them to an appropriate mechanism; and programs for these functions.

### <Flow Channel Device>

The flow channel device is constructed by the cell-spreading substrate and the flow channel-forming member, and a space enclosed by them serves as a flow channel which a liquid such as a cell suspension can be fed to and fill up. From the standpoint of the ease of observation and maintenance, the cell-spreading substrate and the flow channel-forming member may be in a state of being attachable/detachable by a means such as engagement, screw fixation or adhesion. On the ceiling side of the flow channel in the vicinity of the upstream and downstream ends, that is, on the flow channel-forming member, an inlet and an outlet are formed for allowing the above-described various liquids to be fed into or discharged from the flow channel, respectively.

The height of the flow channel (the gap between the cell-spreading substrate and a ceiling member, that is, the thickness of a frame member) is preferably 50 µm to 500 µm. With the height of the flow channel being in this range, since not only the cell suspension (and the cells contained therein) in the flow channel can be easily moved by the force of feeding the liquid but also the flow channel is unlikely to be clogged by the cells, the cells can be spread smoothly.

The flow channel-forming member can be constructed by: a frame member which constitutes the side wall of the flow channel by creating a void that allows the flow channel to have a prescribed height and forming a planar area of the flow channel; and a ceiling member which is arranged on the frame member and constitutes the ceiling of the flow channel. The ceiling member may comprise a space (reservoir) which is connected with the outlet and temporarily retains liquids such as cell suspension. Further, the ceiling part of the flow channel may have an irregular structure such that a flow which allows cells to easily enter the microchambers is generated. The positions of the irregularities can be adjusted in such a manner that, for example, the centers of the microchambers do not align with (are not arranged along the vertical lines of) the centers of recessed parts or the centers of protruded parts and, by positioning the irregularities in this manner, the flow of the cell suspension or the like shifts toward the microchambers at the recessed parts and protruded parts, allowing cells to easily flow into the microchambers.

The cell-spreading substrate and the flow channel-forming member are preferably made of, for example, a transparent material, such as a plastic (e.g., polystyrene, polymethyl methacrylate, polycarbonate or cycloolefin copolymer) or glass (e.g., quartz glass). In a cell analysis system, usually, fluorescently stained cells are observed and photographed; therefore, it is required that at least the part corresponding to the ceiling member of the flow channel-forming member be made of a transparent material so that the fluorescence of specific labeled cells can be measured by the optical detection system of the cell analyzer. Further, the frame member may be made of a material having appropriate elasticity and adhesiveness, such as a silicon resin (e.g., polydimethylsiloxane: PDMS), and the flow channel device may also be constructed by sandwiching this frame member with the cell-spreading substrate and the ceiling member.

### <Reagent Container>

In the reagent container, a variety of liquids that are required to be fed to the flow channel for carrying out the cell detection method, such as a cell suspension, a staining liquid (e.g., a fluorescently labeled antibody solution, a nuclear staining agent solution, or a mixed solution thereof), a washing liquid and, in the second embodiment of the present invention, a nucleic acid amplification reaction reagent and a sealing liquid as well as other required solutions, are stored. For example, a liquid having relatively high storage stability, such as a sealing liquid or a washing liquid, can be stored in a prescribed part of the reagent container in advance in a tightly sealed state, and a liquid required to be prepared immediately prior to cell staining or nucleic acid amplification, such as a cell suspension, a staining liquid or a nucleic acid amplification reaction reagent, can be added to and stored in a prescribed part of the reagent container after being prepared. A solution that is used plural times, such as a washing liquid, may be stored in separate parts in amounts corresponding to the respective steps and, when solutions of the same formulation are repeatedly used in the respective steps, a liquid in a total amount thereof may be stored in a single part. Further, in the reagent container, as required, a part where waste liquids discharged from the flow channel by suction after liquid feeding are retained is arranged.

### [Example 1: Detection by Immunostaining]

### (a) Sample Treatment Step

By density gradient centrifugation utilizing PERCOLL (manufactured by GE Healthcare, specific gravity = 1.113) as a separation medium, erythrocytes were removed from a blood sample collected from a healthy individual. A model sample alternative to a patient sample was prepared by adding to this blood sample about 1,000 PD-L1-positive MIA PaCa-2 cells (pancreas cancer-derived cell strain) and about 10,000 T-cells on which PD-1 was over-expressed. After centrifuging this sample and removing the resulting supernatant, the resultant was allowed to react with a solution, which was obtained by diluting a 20-v/v% formalin solution (manufactured by Wako Pure Chemical Industries, Ltd.; formaldehyde concentration = 8 w/w%) with PBS to a formaldehyde concentration of 4 w/w%, at room temperature for 20 minutes to perform a cell fixation treatment.

### (b) Cell Spreading Step

A flow channel device was assembled using a cell-spreading substrate on which 20,000 microchambers having a diameter of 100 µm and a depth of 50 µm had been formed and a flow channel-forming member capable of forming a flow channel having a height of 0.1 mm and a width of 15 mm with openings. One of the openings was connected to a syringe pump, and a reservoir for adding reagents was formed at the other opening. As a pre-wetting liquid, 40 mL of PBS was extruded from the syringe pump and introduced to the flow channel at a flow rate of 40 mL/min from the side of the suction part to the reservoir side. The thus fixed cell sample was subjected to centrifugal washing with PBS twice immediately before being used and subsequently added to the reservoir, after which 100 µL of the cell sample was introduced to the flow channel at a flow rate of 0.1 mL/min by suction using the syringe pump, whereby cells were stored in the microchambers.

### (c) Staining Step

A solution was prepared by adding a FITC-labeled anti-CD45 antibody (manufactured by Beckman Coulter, Inc.) for specification of leukocytes, a PE-labeled anti-cytokeratin antibody (manufactured by Becton, Dickinson and Co.) for specification of MIA PaCa-2 cells (pancreas cancer-derived cell strain) and Hoechst that binds to cell nuclei, as well as an Alexa Fluor (registered trademark) 647-labeled anti-PD-L1 antibody (CST) and an Alexa Fluor (registered trademark) 750-labeled anti-PD-1 antibody (manufactured by Miltenyi Biotec K.K.), to a PBS solution containing 0.1% of Tween 20 for permeabilization treatment and 3 w/v% of BSA for blocking treatment. The thus prepared solution was introduced from the reservoir by suction using the syringe pump and left to stand for 30 minutes to react with the fixed cells, after which the cells were washed with PBS three times.

### (d) Image Acquisition Step

The resulting stained cells were observed under a fluorescence microscope, and images each corresponding to FITC, PE, Hoechst, Alexa Fluor (registered trademark) 647 and Alexa Fluor (registered trademark) 750 were photographed and integrated together.

### (e) Identification Step

From the photographed images thus integrated in the image acquisition step (d), CD45 (FITC)-negative and cytokeratin (PE)-positive cells with a nucleus (Hoechst) were identified as cancer cells (= MIA PaCa-2 cells), and other cells with a nucleus (Hoechst) were identified as leukocytes.

### (f) Detection Step

Among those cells identified as cancer cells in the identification step (e), PD-L1 (Alexa Fluor (registered trademark) 647)-positive cells were detected as PD-L1-expressing cells, and the number thereof was measured to be 860. Further, among those cells identified as leukocytes, PD-1 (Alexa Fluor (registered trademark) 750)-positive cells were detected as PD-1-expressing cells, and the number thereof was measured to be 9,300. The ratio of the PD-L1 molecule-expressing cells in the cancer cells was 91% (860/950), and the ratio of the PD-1 molecule-expressing cells in the leukocytes was 3% (9,300/310,000).

By presenting the number of the PD-L1-expressing cancer cells and that of the PD-1-expressing leukocytes contained in a certain volume of blood sample in the above-described manner, the effectiveness of a drug, such as an anti-PD-L1 antibody, can be judged. In addition, the ratio of the PD-L1-expressing cancer cells in all of the cancer cells and the ratio of the PD-1-expressing leukocytes in all of the leukocytes can each be presented as well.

### [Example 2: Detection by PCR]

As an embodiment of analyzing information on the expression levels of the molecules of interest on the basis of "the number of microchambers" measured by PCR, the steps (a), (b) and (e) were performed in the same manner as in Example 1, and the steps (c) and (d) were also performed in the same manner as in Example 1 except that the addition of the Alexa Fluor (registered trademark) 647-labeled anti-PD-L1 antibody (CST) and Alexa Fluor (registered trademark) 750-labeled anti-PD-1 antibody (manufactured by Miltenyi Biotec K.K.) was not performed and images corresponding thereto were thus not acquired. Further, the steps (f) and (g) were changed to the following steps (f) and (g'), respectively.

### (f) Nucleic Acid Detection Step

As a nucleic acid amplification reaction reagent, 200 µL of a real-time PCR reagent for amplification of the PD-L1 gene and PD-1 gene (TaqMan Universal Master Mix II, manufactured by Life Technologies Corporation) was prepared. The primers (forward and reverse) and probes had the following base sequences.

### (i) Primers for PD-L1 gene amplification

Forward primer: 5'-GCCGAAGTCATCTGGACAAG-3'
Reverse primer: 5'-TCTCAGTGTGCTGGTCACAT-3'
Probe: 5'-FAM-CACCACCACCAATTCCAAGA-TAMRA-3'

### (ii) Primers for PD-1 gene amplification

Forward primer: 5'-ATAGAACCACAGGGAAGGGG-3'
Reverse primer: 5'-TTATTTAAAGGGGTTGGCCG-3'
Probe: 5'-HEX-GGGTCCTCACCCCCACGTCA-BHQ-2-3'

### (f-1) Nucleic Acid Amplification Reaction Reagent-feeding Step

The above-described reaction reagent in an amount of 200 µL was introduced from the reservoir to the flow channel at a flow rate of 0.1 mL/min by suction using the syringe pump.

### (f-2) Sealing Liquid-feeding Step

From the reservoir side, 200 µL of mineral oil (194836, manufactured by MP Biomedicals, Inc.) was introduced to the flow channel at a flow rate of 0.1 mL/min by suction using the syringe pump.

### (f-3) Nucleic Acid Amplification Reaction Progress Step and Measurement Step

The flow channel device in which cells and the nucleic acid amplification reaction reagent were stored and sealed with the mineral oil in the microchambers was subjected to 40 thermal reaction cycles each consisting of reaction at 95°C for 30 seconds and reaction at 60°C for 1 minute. Thereafter, the fluorescence intensity of FAM and that of HEX were measured for all of the microchambers.

### (f-4) Detection Step

The number of the microchambers containing cancer cells was determined to be 930 in the identification step (e), and the fluorescence emitted by FAM representing the expression of PD-L1 was observed in 840 of the 930 microchambers, which is equivalent to 90%. Further, the number of the microchambers containing leukocytes was determined to be 20,000 in the identification step (e) (the number of cells identified as leukocytes was 270,000), and the fluorescence emitted by HEX representing the expression of PD-1 was observed in 5,700 of the 20,000 microchambers, which is equivalent to 2% of all leukocytes (5,700/270,000).

By presenting the number of the PD-L1-expressing cancer cells and that of the PD-1-expressing leukocytes contained in a certain volume of blood sample in the above-described manner, the effectiveness of a drug, such as an anti-PD-L1 antibody, can be judged. In addition, the ratio of the PD-L1-expressing cancer cells in all of the cancer cells and the ratio of the PD-1-expressing leukocytes in all of the leukocytes can be presented as well.

### [SEQUENCE LISTING FREE TEXT]

SEQ ID NO:1; PD-L1 forward primer
SEQ ID NO:2; PD-L1 reverse primer
SEQ ID NO:3; FAM-modified probe
SEQ ID NO:4; PD-1 forward primer
SEQ ID NO:5; PD-1 reverse primer
SEQ ID NO:6; HEX-modified probe

## Claims

1. A method of simultaneously detecting blood cells, said method comprising the following steps (a) to (f):
(a) removing erythrocytes from blood of a patient (sample treatment step);
(b) adding a prescribed amount of said blood treated in said step (a) to a cell-spreading substrate to arrange first and second target cells on said substrate (cell spreading step);
(c) staining the respective cell markers and nuclear markers of said first and second target cells with phosphors (staining step);
(d) acquiring a fluorescence image of said cell markers and nuclear markers thus stained in said step (c) (image acquisition step);
(e) identifying said first and second target cells from said fluorescence images of said cell markers and nuclear markers thus acquired in said step (d) (identification step); and
(f) detecting cells expressing a specific molecule (molecule A) among those cells identified as said first target cells in said step (e) as well as cells expressing other specific molecule (molecule B), which interacts with said molecule A, among those cells identified as said second target cells in said step (e) (detection step).

2. The method of simultaneously detecting blood cells according to claim 1, wherein
said detection in said step (f) is preformed based on fluorescent staining of said molecules A and B, and
for this purpose, said method further comprises staining said molecules A and B with phosphors in said step (c).

3. The method of simultaneously detecting blood cells according to claim 1 or 2, wherein
a substrate comprising microchambers is used as said cell-spreading substrate, and
said detection in said step (f) is performed based on nucleic acid amplification reactions of the respective genes of said molecules A and B in said microchambers, instead of or in addition to performing said fluorescent staining of said molecules A and B.

4. The method of simultaneously detecting blood cells according to claim 1 or 2, wherein said detection in said step (f) further comprising: acquiring the positional information of said first target cells expressing said molecule A and said second target cells expressing said molecule B; and detecting groups of said cells that are adjacent with each other.

5. The method of simultaneously detecting blood cells according to any one of claims 2 to 4, wherein said method further comprising acquiring fluorescence images of said molecules A and B in said step (d), or acquiring fluorescence images of said cell-spreading substrate in said step (f).

6. The method of simultaneously detecting blood cells according to any one of claims 1 to 5, wherein said method comprises, with the use of a substrate comprising reference points (reticle marks) as said cell-spreading substrate, specifying the position of cells or the position of microchambers on said cell-spreading substrate and subsequently measuring fluorescence.

7. The method of simultaneously detecting blood cells according to any one of claims 1 to 6, wherein said step (f) further comprises acquiring information on the expression level of said molecule A on said first target cells and that of said molecule B on said second target cells.

8. The method of simultaneously detecting blood cells according to any one of claims 1 to 7, wherein a method of removing erythrocytes and granulocytes by density gradient centrifugation is employed in said step (a).

9. The method of simultaneously detecting blood cells according to any one of claims 1 to 8, wherein said method further comprises the step (g) (data presentation step) of presenting data that relate to administration decision of a drug for the treatment or prevention of a disease associated with interactions between said molecules on the basis of at least one information selected from:
(i) information on the ratio of said first target cells expressing said molecule A and that of said second target cells expressing said molecule B, which information is acquired in said step (f);
(ii) positional information of said first target cells expressing said molecule A and said second target cells expressing said molecule B, which information is acquired in said step (f); and
(iii) information on the expression level of said molecule A and that of said molecule B, which information is acquired in said step (f).

10. The method of simultaneously detecting blood cells according to any one of claims 1 to 9, wherein said first target cells are CTCs, and said second target cells are leukocytes.

11. The method of simultaneously detecting blood cells according to claim 10, wherein said molecule A expressed on said CTCs that are said first target cells is PD-L1, and said molecule B expressed on said leukocytes that are second target cells is PD-1.
